(19) Europäisches Patentamt
European Patent Office
Office européen des brevets

(11) Veröffentlichungsnummer: **0 249 229 B1**

(12) **EUROPÄISCHE PATENTSCHRIFT**

(45) Veröffentlichungstag der Patentschrift: **09.09.92**

(51) Int. Cl.$^5$: **H01F 1/37**

(21) Anmeldenummer: **87108449.7**

(22) Anmeldetag: **11.06.87**

Teilanmeldung 91113640.6 eingereicht am 11/06/87.

(54) **Superparamagnetische Feststoffteilchen.**

(30) Priorität: **12.06.86 DE 3619746**

(43) Veröffentlichungstag der Anmeldung:
**16.12.87 Patentblatt 87/51**

(45) Bekanntmachung des Hinweises auf die Patenterteilung:
**09.09.92 Patentblatt 92/37**

(84) Benannte Vertragsstaaten:
**DE FR GB NL**

(56) Entgegenhaltungen:
**EP-A- 0 072 436**
**EP-A- 0 122 055**
**EP-A- 0 176 919**
**BE-A- 532 146**
**FR-A- 1 467 641**
**GB-A- 1 315 392**
**US-A- 3 531 413**
**US-A- 3 917 538**
**US-A- 3 928 220**
**US-A- 4 430 239**

**MAGNETISM AND MAGNETIC MATERIALS, 1972, (18th Annual Conference, Denver), American Institute of Physics, Conf. Proc., Parts I,II, Seiten 966-970, 1973, New York, US, A.E.Berkowitz et al: "Super- paramagnetic ferrite particles produced by milling"**

(73) Patentinhaber: **BASF Aktiengesellschaft**
**Carl-Bosch-Strasse 38**
**W-6700 Ludwigshafen(DE)**

(72) Erfinder: **Mair, Gunther, Dr.**
**Corneliusstrasse 15**
**W-6800 Mannheim 25(DE)**
Erfinder: **Steck, Werner, Dr.**
**Auerstrasse 4**
**W-6700 Ludwigshafen(DE)**

**EP 0 249 229 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue superparamagnetische Feststoffteilchen, die der allgemeinen Formel I

$M_vMn_wZn_xFe_yO_z$ (I)

entsprechen, in welcher für die Variablen folgende Bedingungen gelten:

| | |
|---|---|
| M | Co und/oder Ni |
| v, w | 0 bis 0,998 |
| x | 0,001 bis 0,998 |
| y | 2,001 bis 2,998 |
| z | 3,001 bis 4 |
| v + w + x | 0,002 bis 0,999 |
| v + w + x + y | 3 |
| $v \neq 0$ | wenn w = 0 |
| $w \neq 0$ | wenn v = 0 |

Außerdem betrifft die Erfindung polare und unpolare superparamagnetische Flüssigkeiten, welche mit Tensiden beschichtete superparamagnetische Feststoffteilchen I in flüssigen Medien kolloidal dispergiert enthalten.

Des weiteren betrifft die Erfindung ein neues verbessertes Verfahren zur Herstellung unpolarer superparamagnetischer Flüssigkeiten aus mit anionischen Tensiden beschichteten superparamagnetischen Feststoffteilchen I und unpolaren flüssigen Medien.

Außerdem betrifft die Erfindung ein neues, verbessertes Verfahren zur Herstellung polarer superparamagnetischer Flüssigkeiten aus mit sauren Phosphorsäureestern beschichteten superparamagnetischen Feststoffteilchen I und polaren flüssigen Medien.

Unter Superparamagnetismus ist das ideale weichmagnetische Verhalten eines ferro- oder paramagnetischen Feststoffteilchens zu verstehen. Zu einem solchen Verhalten kommt es, wenn die magnetische Energie K x V eines Feststoffteilchens (K = Anisotropiekonstante, V = Teilchenvolumen) immer kleiner wird und irgendwann die Größenordnung der thermischen Energie k x T (k = Boltzmannsche Konstante, T = absolute Temperatur in Kelvin) erreicht, so daß kein permanenter Dipol mehr vorliegt. Für kubische Ferrite - dieser Verbindungsklasse gehören die erfindungsgemäßen Feststoffteilchen I an - liegt der kritische größte Teilchendurchmesser, ab dem dieses Verhalten auftritt, bei ca. 5 bis 15 nm (vgl. C.P. Bean und J.D. Livingston, "Superparamagnetism", Journal of Applied Physics, Supplement to Volume 30, Number 4, Seiten 120S bis 129S, 1959). Dieser kritische Teilchendurchmesser entspricht im Falle der kubischen Ferrite - unter der Annahme, daß sie als monodisperse, weitgehend porenfreie kugelförmige Teilchen vorliegen - in etwa einer nach Brunauer, Emmet und Teller bestimmten BET-Oberfläche von 40 bis 130 $m^2/g$ (vgl. R. Brdicka, Grundlagen der Physikalischen Chemie, 7. Auflage. VEB-Verlag, Berlin, 1968, Seiten 546 bis 549).

Superparamagnetische Flüssigkeiten sind durch Tenside stabilisierte kolloidale Dispersionen superparamagnetischer Feststoffteilchen in polaren oder unpolaren flüssigen Medien. Im allgemeinen bedecken die Tenside die Oberfläche der superparamagnetischen Feststoffteilchen in Form einer überwiegend monomolekularen Schicht und verhindern so die Sedimentation der superparamagnetischen Feststoffteilchen in einem Schwerefeld, Magnetfeld und/oder in einem elektrischen Feld.

Da der Superparamagnetismus der ursprünglichen Feststoffteilchen weder durch die Tensidbeschichtung noch durch das kolloidale Dispergieren der beschichteten Feststoffteilchen in flüssigen Medien beeinträchtigt wird, zeigen die kolloidalen Dispersionen superparamagnetisches Verhalten. Mit anderen Worten, diese kolloidalen Dispersionen weisen insgesamt ein hysteresisfreies reversibles Magnetisierungs- und Demagnetisierungsverhalten auf.

Im Rahmen der vorliegenden Erfindung bezeichnet der Betriff "flüssiges Medium" die flüssigen molekulardispers miteinander vermischten Bestandteile einer kolloidalen Dispersion tensidbeschichteter superpramagnetischer Feststoffteilchen, wobei ein solches flüssiges Medium in der Hauptsache eine oder mehrere polare oder unpolare Flüssigkeiten wie Wasser, Kohlenwasserstoffe oder Öle enthält und darüber hinaus noch molekulardispers gelöste Zusatzstoffe wie Säuren, Basen, Salze, Gase oder Tenside enthalten kann. Dabei drückt der Begriff "polar" aus, daß die betreffenden Flüssigkeiten und flüssigen Medien in der Lage sind, miteinander oder mit Zusatzstoffen Dipol-Dipol-, Dipol-Ion und/oder Ion-Ion-Wechselwirkungen einzugehen, wogegen der Begriff "unpolar" darauf hindeutet, daß die betreffenden Flüssigkeiten und flüssigen Medien zu solchen Wechselwirkungen nicht in der Lage sind.

Im folgenden werden - der Kürze halber - superparamagnetische Flüssigkeiten auf der Basis unpolarer

flüssiger Medien als "unpolare superparamagnetische Flüssigkeiten" und solche auf der Basis polarer flüssiger Medien als "polare superparamagnetische Flüssigkeiten" bezeichnet.

Im Rahmen der vorliegenden Erfindung werden agglomerierte sedimentierbare superparamagnetische Feststoffteilchen und sedimentierbare magnetische Feststoffteilchen unter dem Begriff "sedimentierbare Feststoffteilchen" zusammengefaßt.

Aus der US-A-3 843 540 ist ein Verfahren zur Herstellung superparamagnetischer Flüssigkeiten aus superparamagnetischen Feststoffteilchen und unpolaren flüssigen Medien bekannt, bei dem man die Teilchen aus wäßrigen Metallsalzlösungen, welche der chemischen Zusammensetzung der Teilchen entsprechen, mittels einer Base ausfällt, mit Tensiden beschichtet, in unpolare Flüssigkeiten überführt, aus diesen mittels Aceton ausflockt, abtrennt und mit Aceton wäscht, um sie anschließend in einer unpolaren Flüssigkeit zu redispergieren.

Das bekannte Verfahren umfaßt nicht die Verwendung superparamagnetischer Feststoffteilchen I und ihr Ausflocken aus ihren Dispersionen in polaren oder unpolaren flüssigen Medien mittels Methanol oder das Waschen von Dispersionen auf der Basis unpolarer flüssiger Medien mit Basen und Wasser.

Aus der US-A-4 430 239 ist ein Verfahren zur Herstellung superparamagnetischer Flüssigkeiten aus superparamagnetischen Magnetitteilchen und polaren flüssigen Medien bekannt, bei dem man die Teilchen aus wäßrigen Metallsalzlösungen mittels einer Base ausfällt, in Wasser dispergiert, mit einem sauren Phosphorsäureester beschichtet, mit Aceton ausflockt, abtrennt und danach in einer polaren Flüssigkeit redispergiert.

Das bekannte Verfahren umfaßt nicht die Verwendung superparamagnetischer Feststoffteilchen I und die Verwendung einer polaren Flüssigkeit 1, welche niedriger siedet als der Hauptbestandteil des polaren flüssigen Mediums der superparamagnetischen Flüssigkeiten, die polare Flüssigkeit 2.

Die bekannten Verfahren weisen den Nachteil auf, daß sie unpolare oder polare superparamagnetische Flüssigkeiten mit einem nichtakzeptablen Anteil an sedimentierbaren Feststoffteilchen liefern, mit der Folge, daß die Sättigungsmagnetisierung $M_s$ der Flüssigkeiten niedriger ist als man aufgrund der Ausgangsstoffmenge erwarten konnte. Außerdem weisen solche superparamagnetischen Flüssigkeiten eine unbefriedigende Stabilität auf, d.h., daß in ihnen im Lauf der Zeit irreversible Vorgänge ablaufen, die zu einer weiteren Bildung von sedimentierbaren Feststoffteilchen führen, wodurch die Anwendbarkeit solcher superparamagnetischer Flüssigkeiten erheblich eingeschränkt, wenn nicht sogar ganz aufgehoben wird. Zwar kann die Bildung sedimentierbarer Feststoffteilchen in einem geringen Umfang durch die Verwendung eines Überschusses an Tensiden, der weit über das hinausgeht, was für die Beschichtung der superparamagnetischen Feststoffteilchen notwendig ist, unterdrückt werden, dann aber erhöht sich bekanntermaßen (vgl. die US-A-3 843 540) die Viskosität der superparamagnetischen Flüssigkeiten in unerwünschter Weise.

Aus der US-A-3 351 413 ist es bekannt, daß sich neben kubischen Ferriten, Magnetit und $\gamma$-$Fe_2O_3$ sowie jedem anderen beliebigen festen magnetischen Material, das in geeigneter Weise zerkleinert werden kann, auch kubische Mangan-Zink-Ferrite allgemein für die Herstellung superparamagnetischer Feststoffteilchen und superparamagnetischer Flüssigkeiten eignen. Es wird in der Patentschrift allerdings nicht angegeben, welche Zusammensetzung, Teilchengröße und Sättigungsmagnetisierung $M_s$ geeignete Mangan-Zink-Ferrite aufweisen sollen.

Gemäß der US-A-4 430 239 kommen für die Herstellung polarer superparamagnetischer Flüssigkeiten neben Magnetit auch andere Ferrite in Betracht. Es wird allerdings nicht angegeben, welche Zusammensetzung diese Ferrite haben sollen.

Üblicherweise aber werden für die Herstellung superparamagnetischer Flüssigkeiten superparamagnetischer Magnetit oder $\gamma$-Ferrit oder Superparamagnetische Mischkristalle aus diesen beiden kubischen Ferriten verwendet (vgl. die US-A-3 843 540), wobei es allgemein bekannt ist, daß die Sättigungsmagnetisierung $M_s$ durch die Mischkristallbildung gegenüber den reinen Ferriten in geringem Umfange verbessert werden kann.

Aus R.S. Tebble und D.J. Craik, "Magnetic Materials", Wiley-Interscience, London, Seiten 252 bis 270, 1969 ist die Sättigungsmagnetisierung $M_s$ von nicht superparamagnetischen Magnetit-, $MnFe_2O_4$-, $CoFe_2O_4$ und $NiFe_2O_4$-Feststoffteilchen bekannt (vgl. insbesondere Seite 256, Figur 7.2). Außerdem geht insbesondere aus der Seite 266, Figur 7.10, hervor, daß die Sättigungsmagnetisierung $M_s$ solcher kubischer Ferrite durch die Substitution eines Teils der Mangan-, Kobalt- oder Nickel-Ionen durch Zink-Ionen erhöht werden kann. Dabei wird bei einer Zusammensetzung, die der allgemeinen Formel II

$M_w^1Zn_xFe_2O_4$ (II)

mit den Variablen

$M^1$ Co, Ni oder Mn

w 0,4 bis 0,5
x 0,5 bis 0,6
w + x 1

entspricht, jeweils ein Maximum der Sättigungsmagnetisierung $M_s$ erreicht.

Superparamagnetische Ferrite bekannter Zusammensetzung weisen aber den Nachteil auf, daß ihre Sättigungsmagnetisierung $M_s$ vergleichsweise niedrig ist. Dem entsprechend müssen für die Herstellung superparamagnetischer Flüssigkeiten hoher Sättigungsmagnetisierung $M_s$ jeweils große Mengen an Ferriten verwendet werden. Die betreffenden superparamagnetischen Flüssigkeiten sind dann wegen ihres notwendigerweise hohen Feststoffgehalts unerwünscht hochviskos und oftmals instabil und daher für viele Anwendungszwecke nur bedingt geeignet.

Aufgabe der vorliegenden Erfindung war es, neue superparamagnetische Feststoffteilchen und Flüssigkeiten mit verbesserten anwendungstechnischen Eigenschaften sowie ein neues verbessertes Verfahren zur Herstellung solcher superparamagnetischer Flüssigkeiten zu finden.

Demgemäß wurden die eingangs definierten superparamagnetischen Feststoffteilchen I gefunden.

Außerdem wurden superparamagnetische Flüssigkeiten, welche mit Tensiden beschichtete superparamagnetische Feststoffteilchen I in flüssigen Medien kolloidal dispergiert enthalten, gefunden.

Des weiteren wurde ein Verfahren zur Herstellung unpolarer superparamagnetischer Flüssigkeiten aus superparamagnetischen Feststoffteilchen I und unpolaren flüssigen Medien gefunden, bei dem man die Teilchen aus wäßrigen Metallsalzlösungen, die der chemischen Zusammensetzung der Teilchen entsprechen, mittels einer Base ausfällt, mit anionischen Tensiden beschichtet und in eine unpolare Flüssigkeit überführt, wobei das Verfahren dadurch gekennzeichnet ist, daß man

a) die in dem wäßrigen Medium befindlichen beschichteten Teilchen I mit Methanol ausflockt, abtrennt und in einer unpolaren Flüssigkeit redispergiert

und/oder

b) die bereits auf beliebige Weise in einem unpolaren flüssigen Medium dispergierten beschichteten Teilchen I mit Methanol ausflockt, abtrennt und in einer unpolaren Flüssigkeit redispergiert.

In einer bevorzugten Ausführungsform des erfindungsgemäßen Verfahrens wird der Verfahrensschritt b zwei- oder mehrmals wiederholt.

In einer weiteren bevorzugten Ausführungsform werden die in erfindungsgemäßer Weise erhaltenen Dispersionen zunächst mit einer wäßrigen Base und danach solange mit Wasser gewaschen, bis das Waschwasser neutral reagiert und keine Anionen damit mehr nachweisbar sind.

Zudem wurde ein Verfahren zur Herstellung polarer superparamagnetischer Flüssigkeiten aus superparamagnetischen Feststoffteilchen I und polaren flüssigen Medien gefunden, bei dem man die Teilchen aus wäßrigen Metallsalzlösungen, die der chemischen Zusammensetzung der Teilchen entsprechen, mittels einer Base ausfällt, mit sauren Phosphorsäureestern beschichtet und aus dem wäßrigen Medium in eine polare Flüssigkeit, die höher siedet als Wasser, überführt, wobei das Verfahren dadurch gekennzeichnet ist, daß man hierbei

a') die beschichteten superparamagnetischen Feststoffteilchen I in eine zwischen 110 und 250 °C siedende polare Flüssigkeit 1 überführt und daß man

b') die resultierende Dispersion nach dem Abtrennen des Wassers mit einer polaren Flüssigkeit 2 versetzt, welche höher siedet als die Flüssigkeit 1, wonach man die Flüssigkeit 1 aus der Dispersion entfernt.

Die erfindungsgemäßen superparamagnetischen Feststoffteilchen I gehören der ihnen übergeordneten Verbindungsklasse der kubischen Ferrite an. Sie sind demnach als superparamagnetische modifizierte kubische Ferrite und spezieller als superparamagnetische modifizierte kubische Zink-Eisen-Ferrite anzusehen, deren Sättigungsmagnetisierung $M_s$ durch Modifizierung, d.h. durch Einbau von Kobalt-, Nickel- und/oder Manganionen in das Ferritgitter, erhöht worden ist. Die erfindungsgemäßen superparamagnetischen Feststoffteilchen I sind daher als kubische

Kobalt-Zink-Eisen-Ferrite Ia,
Nickel-Zink-Eisen-Ferrite Ib,
Kobalt-Nickel-Zink-Eisen-Ferrite Ic,
Mangan-Zink-Eisen-Ferrite Id,
Kobalt-Mangan-Zink-Eisen-Ferrite Ie,
Nickel-Mangan-Zink-Eisen-Ferrite If und als
Kobalt-Nickel-Mangan-Zink-Eisen-Ferrite Ig

zu bezeichnen.

Beispiele für erfindungsgemäße superparamagnetische Feststoffteilchen I, für deren Zusammensetzung die Bedingung w = 0 gilt, sind:

Ia) superparamagnetische kubische Kobalt-Zink-Eisenferrite wie
$Co_{0,5}Zn_{0,2}Fe_{2,3}O_{3,7}$ oder
$Co_{0,3}Zn_{0,1}Fe_{2,6}O_{3,84}$;
Ib) superparamagnetische kubische Nickel-Zink-Eisenferrite wie
$Ni_{0,4}Zn_{0,4}Fe_{2,2}O_{3,5}$,
$Ni_{0,01}Zn_{0,05}Fe_{2,94}O_{3,95}$ oder
$Ni_{0,5}Zn_{0,1}Fe_{2,4}O_{3,8}$;
und
Ic) superparamagnetische kubische Kobalt-Nickel-Zink-Eisenferrite wie
$Co_{0,1}Ni_{0,3}Zn_{0,4}Fe_{2,2}O_{4}$;
$Co_{0,3}Ni_{0,01}Zn_{0,27}Fe_{2,42}O_{3,6}$ oder
$Co_{0,1}Ni_{0,1}Zn_{0,7}Fe_{2,1}O_{3,9}$.

Beispiele für erfindungsgemäße superparamagnetische Feststoffteilchen I, für deren Zusammensetzung die Bedingung v = 0 gilt, sind

Id) superparamagnetische kubische Mangan-Zink-Eisenferrite wie
$Mn_{0,5}Zn_{0,2}Fe_{2,3}O_{3,9}$;
$Mn_{0,2}Zn_{0,3}Fe_{2,5}O_{3,95}$,
$Mn_{0,1}Zn_{0,1}Fe_{2,8}O_{4}$ oder
$Mn_{0,02}Zn_{0,05}Fe_{2,93}O_{3,8}$.

Beispiele für erfindungsgemäße superparamagnetische Feststoffteilchen I, für deren Zusammensetzung die Bedingungen v ≠ 0 und w ≠ 0 gelten - letztere ergeben sich unmittelbar aus den Bedingungen für die Variablen nach Anspruch 1 - sind

Ie) superparamagnetische kubische Kobalt-Mangan-Zink-Eisen-Ferrite wie
$Co_{0,1}Mn_{0,1}Zn_{0,35}Fe_{2,45}O_{3,9}$ oder
$Co_{0,6}Mn_{0,1}Zn_{0,2}Fe_{2,1}O_{4}$;
If) superparamagnetische kubische Nickel-Mangan-Zink-Eisen-Ferrite wie
$Ni_{0,3}Mn_{0,1}Zn_{0,1}Fe_{2,5}O_{3,8}$ oder
$Ni_{0,01}Mn_{0,01}Zn_{0,2}Fe_{2,78}O_{4}$;
und
Ig) superparamagnetische kubische Kobalt-Nickel-Mangan-Zink-Eisen-Ferrite wie
$Co_{0,1}Ni_{0,15}Mn_{0,2}Zn_{0,1}Fe_{2,45}O_{3,9}$,
$Co_{0,01}Ni_{0,22}Mn_{0,01}Zn_{0,002}Fe_{2,958}O_{4}$ oder
$Co_{0,34}Ni_{0,2}Mn_{0,31}Zn_{0,1}Fe_{2,05}O_{3,72}$.

Von diesen erfindungsgemäßen superparamagnetischen Feststoffteilchen Ia bis Ig sind die superparamagnetischen kubischen Kobalt-Mangan-Zink-Eisen-Ferrite Ie und die superparamagnetischen kubischen Nickel-Mangan-Zink-Eisen-Ferrite If besonders bevorzugt.

Die Teilchengröße der Feststoffteilchen I entspricht einer inneren Oberfläche nach BET von 40 bis 130, vorzugsweise 50 bis 120 und insbesondere 60 bis 110 g/m$^2$.

In einem Magnetfeld der Stärke von 160 kA/m weisen die Feststoffteilchen I eine Sättigungsmagnetisierung $M_s$ von über 60 nTm$^3$/g auf.

Die erfindungsgemäßen superparamagnetischen Feststoffteilchen I können nach beliebigen geeigneten Methoden, beispielsweise durch Zerkleinern größerer, nicht superparamagnetischer Feststoffteilchen entsprechender Zusammensetzung, erhalten werden. Vorteilhafterweise erfolgt jedoch ihre Herstellung durch rasches Ausfällen aus wäßrigen lösungen, in denen die Kobalt-, Nickel- und/oder Mangansalze sowie die Zink und Eisensalze in den entsprechenden Kombinationen und molaren Verhältnissen vorliegen, durch Zugabe in etwa stöchiometrischer Mengen an wäßrigen Basen wie Natronlauge. Dabei ist es üblich, das Ausfällen unter Inertgas durchzuführen. Es ist von Vorteil, wenn das Verhältnis q von Gehalt an zweiwertigem Eisen zu Gesamteisengehalt in den wäßrigen lösungen vor dem Ausfällen q = 0,01 bis 0,9, vorzugsweise 0,15 bis 0,6 und insbesondere 0,2 bis 0,5 beträgt.

Nach dem Ausfällen wird in den Reaktionsgemischen aus Feststoffteilchen I und wäßrigen Medien in üblicher und bekannter Weise ein pH von 10 bis 12, vorzugsweise 11, eingestellt, wonach man die Reaktionsgemische bei Raumtemperatur eine gewisse Zeit stehen läßt. Anschließend werden die Reaktionsgemische neutralisiert. Danach trennt man die Feststoffteilchen I in üblicher und bekannter Weise ab, wäscht sie nach und trocknet sie.

Die Feststoffteilchen I eignen sich hervorragend als Komponenten von magnetischen Tonern und Tinten. Vor allem aber eignen sie sich als superparamagnetische Komponenten in den erfindungsgemäßen superparamagnetischen Flüssigkeiten.

Die erfindungsgemäßen superparamagnetischen Flüssigkeiten enthalten die mit Tensiden beschichteten

erfindungsgemäßen superparamagnetischen Feststoffteilchen I kolloidal dispergiert in flüssigen Medien.

Bei diesen flüssigen Medien kann es sich sowohl um polare als auch um unpolare Medien handeln, wobei die polaren Medien in der Hauptsache polare Flüssigkeiten und die unpolaren Medien in der Hauptsache unpolare Flüssigkeiten enthalten.

Eine geeignete polare Flüssigkeit ist Wasser.

Beispiele geeigneter polarer Flüssigkeiten, welche höher sieden als Wasser, sind Ether wie Diethylenglykoldimethylether, Diethylenglykoldiethylether oder Diethylenglykolmonomethylether; $C_2$-$C_6$-Alkylester von $C_1$-$C_6$-Alkanmonocarbonsäuren, wie Essigsäurehexylester, Essigsäurecyclohexylester, Propionsäurepentylester, Buttersäureethylester, Pentancarbonsäuremethylester, Pentancarbonsäureethylester oder Hexancarbonsäuremethylester; oder $C_5$-$C_6$-Dialkylester von $C_1$-$C_7$-Alkandicarbonsäuren wie Malonsäuredihexylester, Bernsteinsäuredihexylester, Glutarsäuredipentylester, Adipinsäuredi-n-butylester, Pimelinsäuredi-n-butylester, Hexandicarbonsäuredi-n-propylester oder Azelainsäurediethylester (Heptandicarbonsäurediethylester). Polare Flüssigkeiten dieser Art sieden zwischen 110 und 250 °C. Sie kommen als polare Flüssigkeiten 1 in Betracht. Von Vorteil ist der Essigsäurehexyl-und der Essigsäurecyclohexylester.

Beispiele weiterer geeigneter polarer Flüssigkeiten, welche höher sieden als die vorstehend genannten, sind höhere Dialkylester von Alkandicarbonsäuren wie Adipinsäuredinonylester, Adipinsäuredidecylester oder Adipinsäurediisodecylester, Hexandicarbonsäure-n-octylester, Azelainsäuredi(2-ethylhexyl)-ester oder Azelainsäuredi-n-octylester; höhere Dialkylester der Phthalsäure, Isophthalsäure oder Terephthalsäure wie Phthalsäuredinonylester, Phthalsäuredidecylester, Phthalsäurediisodecylester, Phthalsäureundecylester, Isophthalsäurediisodecylester oder Terephthalsäuredi-(2-ethylhexylester), höhere Trialkylester der Trimellithsäure wie Trimellithsäuretri-n-octylester oder Alkylbenzylester der Phthalsäure wie Phthalsäure-n-octylbenzylester. Polare Flüssigkeiten dieser Art sieden im allgemeinen oberhalb 250 °C. Sie kommen als polare Flüssigkeiten 2 in Betracht. Von Vorteil sind der Phthalsäurediisodecylester, der Adipinsäuredinonylester und der Adipinsäurediisodecylester.

Beispiele geeigneter unpolarer Flüssigkeiten sind aliphatische Kohlenwasserstoffe wie Hexane, Heptane, Octane, Nonane, Cyclopentan, Alkylcyclopentane, Cyclohexan, Alkylcyclohexane oder Petrolether eines Siedebereiches von 20 bis 160 °C; aromatische Kohlenwasserstoffe wie Benzol oder Toluol; oder halogenierte aliphatische Kohlenwasserstoffe wie Methylenchlorid, Chloroform, Dichlordifluormethan oder die Dichlortetrafluorethane; von denen die Petrolether besonders vorteilhaft sind.

Weitere Beispiele geeigneter unpolarer Flüssigkeiten sind Mineralöle, Silikonöle, Öle auf der Basis von fluorierten Ethern, von fluorierten aliphatischen Kohlenwasserstoffen, von Alkylestern von Mono- und/oder Dicarbonsäuren, von hydrierten Poly-$\alpha$-olefinen, von Polyisobutylen, von Alkylaromaten, von hydrierten Poly-$\alpha$-olefinen und Alkylaromaten oder von Gemische aus solchen Ölen, wobei die Öle auf der Basis von hydrierten Poly-$\alpha$-olefinen und/oder von Alkylaromaten sowie Öle auf der Basis von Polyisobutylen und die aliphatischen Mineralöle von besonderem Vorteil sind.

Die Viskosität geeigneter Öle liegt im allgemeinen bei 2 bis 1000 mPas bei beispielsweise 20 °C und/oder bei 1 bis 300 mPas bei beispielsweise 40 °C. Im allgemeinen liegt ihre Verdampfungsrate bei 80 °C unterhalb von $10^{-5}$, vorzugsweise $10^{-7}$ und insbesondere $10^{-8}$ $gcm^{-2}s^{-1}$. Sie weisen oftmals eine Dichte bei 20 °C von 0,7 bis 0,9 $gcm^{-3}$ auf. Ihr Siedebereich liegt vorzugsweise bei 200 bis 600 °C bei Normaldruck, bei 0,01 bis 0,3 mbar kann der Siedebereich auch bei 100 bis 300 °C liegen.

Für die Beschichtung der Feststoffteilchen I kommen vor allem wasserlösliche kationische und anionische Tenside oder elektrisch neutrale Tenside wie Ethylenoxid-Propylenoxid-Blockcopolymerisate in Frage; bevorzugt sind die anionischen Tenside.

Beispiele geeigneter anionischer Tenside sind demnach Alkylsulfonate und ihre Salze wie etwa Natriumeicosanylsulfonat oder Kaliumperfluoroctadecylsulfonat; Fettalkoholethersulfate und ihre Salze wie etwa Natrium-3,6-dioxaoctadecylsulfat;Fettalkoholphosphorsäuremonoester und ihre Mono- und Dialkalisalze wie etwa Natrium- und Dinatriumoctadecylphosphat; 2-Ethylhexylphosphat, Di-n-octylphosphat oder Oleylphosphat; Phosphorsäuremono- und -diester von alkoxylierten Alkoholen der allgemeinen Formel III

$$R^1\text{-}O\text{-}(R^2\text{-}O)_{\overline{p}}\text{-}H \qquad III$$

worin $R^1$ einen $C_6$-$C_{18}$-Alkylrest oder einen $C_{10}$-$C_{20}$-Arylalkylrest, $R^2$ einen $C_2$-$C_4$-Alkandiylrest und p eine Zahl von 1 bis 15 bezeichnet, wie Phosphorsäuremono- und -diester von ethoxyliertem n-Octylalkohol, ethoxyliertem Nonylalkohol, ethoxyliertem Decylalkohol, ethoxyliertem Pentadecylalkohol, propoxyliertem Octadecylalkohol, ethoxyliertem $\omega$-Phenylnonylalkohol, ethoxyliertem 2-Ethylhexanol, propoxyliertem 2-Ethylhexanol, ethoxyliertem 7-(p-Pentylphenyl)-heptylalkohol oder ethoxyliertem $\omega$-Phenyl decylalkohol oder

Gemische aus solchen Estern; oder langkettige, einfach oder mehrfach olefinisch ungesättigte Fettsäuren mit 16 bis 25 Kohlenstoffatomen im Alkenylrest und ihre Alkali-oder Ammoniumsalze wie etwa Ölsäure oder Natrium- oder Ammoniumoleat.

Bevorzugt sind die Ölsäure und ihre Salze sowie die Phosphorsäuremono- und -diester von alkoxylierten Alkoholen der allgemeinen Formel III, worin $R^1$ einen $C_{12}$-$C_{17}$-Arylalkylrest, $R^2$ einen Ethylenrest und p eine Zahl von 1 bis 5 bezeichnet, wobei Natriumoleat und der Phosphorsäuremonoester von ethoxyliertem (p = 3 bis 5) ω-Phenylnonylalkohol ganz besonders bevorzugt sind.

Die Herstellung der erfindungsgemäßen polaren und unpolaren superparamagnetischen Flüssigkeiten kann in geeigneter Weise nach Methoden, die vom Stand der Technik her bekannt sind, erfolgen.

Es ist jedoch von besonderem Vorteil, wenn die erfindungsgemäßen unpolaren superparamagnetischen Flüssigkeiten nach dem erfindungsgemäßen Verfahren zur Herstellung unpolarer superparamagnetischer Flüssigkeiten und die erfindungsgemäßen polaren superparamagnetischen Flüssigkeiten nach dem erfindungsgemäßen Verfahren zur Herstellung polarer Superparamagnetischer Flüssigkeiten gewonnen werden.

Beispiele für erfindungsgemäß geeignete unpolare Flüssigkeiten sind die vorstehend genannten, von denen die Petrolether, die Öle auf der Basis von hydrierten Poly-α-olefinen und/oder von Akylaromaten sowie die Öle auf der Basis von Polyisobutylen und die aliphatischen Mineralöle von besonderem Vorteil sind.

Beispiele für erfindungsgemäß geeignete anionische Tenside sind die vorstehend genannten, von denen die Ölsäure und ihre Salze von besonderem Vorteil sind.

Verfahrenstechnisch bietet das Verfahren keine Besonderheiten - d.h., daß zu seiner Ausübung an und für sich keine speziell hierfür entwickelte und angepaßte Apparaturen notwendig sind und die einzelnen Verfahrensschritte - jeder für sich gesehen - auf bekannten chemischen Arbeitsmethoden beruhen.

Das erfindungsgemäße Verfahren geht aus von der Herstellung kolloidaler wäßriger Dispersionen von mit anionischen Tensiden beschichteten superparamagnetischen Feststoffteilchen I.

Im allgemeinen geschieht dies durch rasches Ausfallen der erfindungsgemäßen superparamagnetischen Feststoffteilchen I unter Inertgas aus wäßrigen Lösung entsprechend zusammengesetzter Salzgemische mittels in etwa stöchiometrischer Mengen an wäßrigen Basen wie etwa Natronlauge. Dabei kann die Teilchengröße der Feststoffteilchen I durch Variation der Salz- und Basenkonzentration, der Fällungstemperatur- und -geschwindigkeit sowie des Verhältnisses q von Gehalt an zweiwertigem Eisen zu Gesamteisengehalt in den wäßrigen Lösungen vor dem Ausfällen in gewünschter Weise eingestellt werden. Üblicherweise wird in den Reaktionsgemischen aus Feststoffteilchen I und wäßrigen Medien nach dem Ausfällen ein pH von 10 bis 12, vorzugsweise 11, eingestellt, wonach man die Reaktionsgemischen eine gewisse Zeit, üblich sind 15 bis 60 Minuten, bei Raumtemperatur stehen läßt. Danach werden die Reaktionsgemische im allgemeinen neutralisiert. Die Feststoffteilchen I werden nun in geeigneter Weise von den wäßrigen Medien abgetrennt, beispielsweise durch Sedimentation in einem Magnetfeld oder durch Filtration, und weitgehend elektrolytfrei gewaschen. Danach werden die Feststoffteilchen I, oder deren Aufschlämmungen in Wasser mit wäßrigen Lösungen eines oder mehrerer anionischer Tenside, beispielsweise mit wäßrigen Lösungen von Natriumoleat, versetzt, wobei man üblicherweise einen Überschuß an Tensiden verwendet, der über die Menge hinausgeht, wie sie normalerweise zur Bedeckung der erfindungsgemäßen superparamagnetischen Feststoffteilchen I mit einer monomolekularen Schicht notwendig ist. Die so erhaltenen kolloidalen Dispersionen aus beschichteten Teilchen I und wäßrigen Medien werden auf einen pH von 10 bis 12, vorzugsweise 11, eingestellt und längere Zeit, üblich sind 15 Minuten bis 3 Stunden, auf Temperaturen von 70 bis 100 ° C, vorzugsweise 90 ° C, unter Rühren erhitzt und gegebenenfalls anschließend neutralisiert.

Es ist üblich diesen Verfahrensschritt insgesamt unter Ausschluß von Luftsauerstoff durchzuführen.

Die in den wäßrigen Medien befindlichen beschichteten Teilchen I werden nun in dem erfindungsgemäßen Verfahrensschritt a durch Zugabe von Methanol ausgeflockt, wobei die Methanolvolumina den Volumina der wäßrigen Medien in etwa entsprechen. Von Vorteil ist es, wenn, bezogen auf die Volumina der wäßrigen Medien, ein Methanolüberschuß verwendet wird.

Die ausgeflockten beschichteten Teilchen I werden von den polaren flüssigen Medien, die in der Hauptsache Wasser und Methanol enthalten, in bekannter Weise, z.B. durch Zentrifugieren und/oder durch Sedimentation in einem Magnetfeld, abgetrennt und gegebenenfalls mit frischem Methanol gewaschen und/oder getrocknet. Danach werden die Teilchen I in unpolaren Flüssigkeiten redispergiert, wobei es erfindungsgemäß von Vorteil ist, wenn man sie zunächst in unpolaren Flüssigkeiten wie Petrolether, welche niedriger sieden als die erfindungsgemäß anzuwendenden Öle, redispergiert.

Die in den unpolaren flüssigen Medien redispergierten beschichteten Teilchen I werden nun in dem erfindungsgemäßen Verfahrensschritt b erneut mittels Methanol ausgeflockt, wobei die Methanolvolumina den Volumina der unpolaren flüssigen Medien in etwa entsprechen. Von Vorteil ist es, wenn, bezogen auf

die Volumina der unpolaren flüssigen Medien, ein Methanolüberschuß verwendet wird.

Die ausgeflockten beschichteten Teilchen I werden von den unpolaren flüssigen Medien abgetrennt, gegebenenfalls mit frischem Methanol gewaschen und/oder getrocknet und anschließend in unpolaren Flüssigkeiten redispergiert. Dabei ist es erfindungsgemäß von Vorteil, die ausgeflockten beschichteten Teilchen I in niedriger siedenden unpolaren Flüssigkeiten wie Petrolether zu redispergieren, wobei die Volumina der unpolaren Flüssigkeiten in etwa den Volumina der ursprünglichen unpolaren flüssigen Medien entsprechen.

Der erfindungsgemäße Verfahrensschritt b kann zwei- oder mehrmals durchgeführt werden.

Für den Fall, daß der erfindungsgemäße Verfahrensschritt a entfällt, erfolgt das Überführen der in den wäßrigen Medien befindlichen beschichteten Teilchen I in die unpolaren flüssigen Medien bekanntermaßen durch Zugabe unpolarer Flüssigkeiten, Durchmischen der resultierenden polaren und unpolaren flüssigen Mischmedien und Phasentrennung, wonach man den erfindungsgemäßen Verfahrensschritt b obligatorisch zwei- oder mehrmals durchführt.

Die hiernach erhaltenen Dispersionen aus beschichteten Teilchen I und unpolaren flüssigen Medien können zunächst mit einer wäßrigen Base wie Natronlauge, Kalilauge oder Ammoniakwasser und danach mit Wasser so lange gewaschen werden, bis das Waschwasser neutral reagiert und keine Anionen darin mehr nachweisbar sind, d.h., daß übliche und bekannte Nachweisreaktionen für die betreffenden Anionen negativ verlaufen.

Dabei kann sowohl die Wäsche mit Basen als auch die mit Wasser kontinuierlich in hierfür geeigneten Anlagen, beispielsweise in Extraktionskolonnen oder Gegenstromextraktoren, oder diskontinuierlich, beispielsweise in Scheidetrichtern oder Rührkesseln, durchgeführt werden. Es versteht sich von selbst, daß nach jedem Waschschritt eine Phasentrennung erfolgt. Wird das Waschen diskontinuierlich durchgeführt, dann ist es von Vorteil, jeweils mehrere kleine Portionen an wäßrigen Basen und Wasser anstelle jeweils einer großen Portion zu verwenden.

Im Rahmen des erfindungsgemäßen Verfahrens ist das Waschen mit wäßrigen Basen und mit Wasser dann obligatorisch, wenn die beiden Verfahrensschritte a und b nicht ausgeführt werden.

Die in dieser Weise erhaltenen erfindungsgemäßen superparamagnetischen Flüssigkeiten können, falls sich dies als notwendig erweist, noch in geeigneter Weise getrocknet werden. In Frage kommt hierbei beispielsweise die azeotrope Destillation des Wassers aus den unpolaren flüssigen Medien nach Zusatz von Verbindungen, die mit Wasser azeotrop siedende Gemische bilden.

Aus den nach dem erfindungsgemäßen Verfahren hergestellten erfindungsgemäßen Dispersionen aus beschichteten superparamagnetischen Feststoffteilchen I und unpolaren flüssigen Medien können die Teilchen I in andere unpolare flüssige Medien überführt werden. Dies kann z.B. dadurch geschehen, daß man in dem erfindungsgemäßen Verfahrensschritt b die ausgeflockten beschichteten Teilchen I in einer anderen als der vor dem Ausflocken verwendeten unpolaren Flüssigkeit redispergiert. Dabei kann man die Menge der zweiten unpolaren Flüssigkeit so wählen, daß erfindungsgemäße superparamagnetischen Flüssigkeiten mit einem gewünschten Teilchengehalt resultieren. Bevorzugt wird jedoch die zweite unpolare Flüssigkeit zu der nach dem erfindungsgemäßen Verfahren erhaltenen erfindungsgemäßen superparamagnetischen Flüssigkeit hinzugegeben, wobei es von Vorteil ist, wenn die zweite unpolare Flüssigkeit sehr viel höher siedet als diejenige, welche die Basis des flüssigen unpolaren Mediums der erfindungsgemäßen superparamagnetischen Flüssigkeit bildet, und wobei ihre Menge so gewählt wird, daß die neue Dispersion nach dem Abdampfen der niedriger siedenden unpolaren Flüssigkeit aus dem Mischmedium den gewünschten Teilchengehalt aufweist. Als niedriger siedende unpolare Flüssigkeiten kommen hier vor allem Petrolether in Frage. Beispiele geeigneter höher siedender unpolarer Flüssigkeiten sind die vorstehend genannten Öle.

Das erfindungsgemäße Verfahren zur Herstellung erfindungsgemäßer polarer Superparamagnetischer Flüssigkeiten geht gleichfalls von der Herstellung der erfindungsgemäßen superparamagnetischen Feststoffteilchen I aus, wobei es von sauren Phosphorsäureestern sowie von polaren Flüssigkeiten 1 und 2 Gebrauch macht.

Beispiele für erfindungsgemäß geeignete saure Phosphorsäureester sind die vorstehend genannten Phosphorsäuremono- und -diester, von denen der Phosphorsäuremonoester des ethoxylierten (p = 3 bis 5) ω-Phenylnonylalkohols von besonderem Vorteil ist.

Beispiele für erfindungsgemäß geeignete polare Flüssigkeiten 1 sind die vorstehend genannten polaren Flüssigkeiten, welche zwischen 110 und 250 °C sieden. Von diesen ist der Essigsäurehexyl- oder der Essigsäurecyclohexylester von ganz besonderem Vorteil.

Beispiele für erfindungsgemäß geeignete polare Flüssigkeiten 2 sind die vorstehend genannten polaren Flüssigkeiten, welche oberhalb 250 °C sieden. Besonders vorteilhaft snd hierbei der Phthalsäurediisodecylester, der Adipinsäuredinonylester und der Adipinsäurediisodecylester.

Auch dieses erfindungsgemäße Verfahren bietet keine methodischen Besonderheiten - d.h. daß zu seiner Ausübung an und für sich keine speziell hierfür entwickelte und angepaßte Apparaturen notwendig sind und die einzelnen Verfahrensschritte - jeder für sich gesehen - auf bekannten chemischen Arbeitsmethoden beruhen.

Dabei erfolgt die Herstellung der wäßrigen Dispersionen von beschichteten Feststoffteilchen I wie vorstehend bei dem Verfahren zur Herstellung erfindungsgemäßer unpolarer superparamagnetischer Flüssigkeiten beschrieben, nur daß anstelle von beispielsweise Ölsäure die sauren Phosphorsäureester für die Beschichtung verwendet werden.

Abweichend von diesem Verfahren, werden nun zu den wäßrigen Dispersionen der mit sauren Phosphorsäureestern beschichteten Feststoffteilchen I im Verfahrensschritt a' polare Flüssigkeiten 1 hinzugegeben, wobei deren Flüssigkeitsvolumina den Volumina der wäßrigen Medien in etwa entsprechen.

Es ist indes auch möglich, zunächst die Feststoffteilchen I herzustellen, zu isolieren und in einem Gemisch aus Wasser, sauren Phosphorsäureestern und polaren Flüssigkeiten 1 zu redispergieren.

Anschließend wird unter Rühren die Temperatur der Dispersionen auf 160 bis 180 ° C erhöht, und das darin enthaltene Wasser wird abdestilliert. Danach werden die wasserfreien Dispersionen während einer gewissen Zeit, beispielsweise während 30 Minuten, bei 160 bis 180 ° C nachgerührt.

Nach dem Abkühlen werden die Dispersionen aus den beschichteten Feststoffteilchen I in den polaren Flüssigkeiten 1 im Verfahrensschritt b' mit der gewünschten Menge an polaren Flüssigkeiten 2 versetzt. Hiernach werden die Flüssigkeiten 1 im Vakuum abdestilliert, und es resultieren die erfindungsgemäßen polaren superparamagnetischen Flüssigkeiten.

Es ist von Vorteil, dieses Verfahren unter Inertgas auszuführen.

Darüber hinaus können die beiden erfindungsgemäßen Verfahren weitere Verfahrensschritte umfassen. So kann es sich als vorteilhaft erweisen, wenn man nach den erfindungsgemäßen Verfahrensschritten aus den dann jeweils vorliegenden Dispersionen aus beschichteten Teilchen I und flüssigen Medien die sedimentierbaren Feststoffteilchen durch Zentrifugieren und/oder durch Sedimentieren in einem Magnetfeld abtrennt, wobei im allgemeinen dem Zentrifugieren der Vorzug gegeben wird.

Obwohl das Zentrifugieren im Rahmen des erfindungsgemäßen Verfahrens an sich nicht ausgeübt werden muß, wird es dennoch immer ausgeführt, da es sich hervorragend dazu eignet, die Vorteile, durch welche sich die erfindungsgemäßen Verfahren auszeichnen, zu belegen. Da beim Zentrifugieren den Dispersionen die sedimentierbaren Feststoffteilchen entzogen werden, erniedrigt sich die Sättigungsmagnetisierung $M_s$ von Dispersionen mit einem hohen Anteil an sedimentierbaren Feststoffteilchen gegenüber der Sättigungsmagnetisierung $M_s$ von vergleichbaren Dispersionen mit einem niedrigen Anteil an solchen Teilchen. Geht man also bei der Herstellung der betreffenden Dispersionen nach unterschiedlichen Verfahren vor, aber von den gleichen Ausgangsstoffen in den gleichen Mengen aus, so wird die unterschiedliche Sättigungsmagnetisierung $M_s$ der betreffenden Dispersionen, d.h. der superparamagnetischen Flüssigkeiten, zu einem direkten Maß des Erfolgs der unterschiedlichen Verfahren.

Die erfindungsgemäßen Verfahren weisen gegenüber dem Stand der Technik zahlreiche Vorteile auf. Mit ihrer Hilfe lassen sich die erfindungsgemäßen unpolaren und polaren superparamagnetischen Flüssigkeiten in einfacher und exakt reproduzierbarer Weise herstellen.

Insgesamt sind die erfindungsgemäßen polaren und unpolaren superparamagnetischen Flüssigkeiten auf der Basis der Feststoffteilchen I und insbesondere die nach den erfindungsgemäßen Verfahren hergestellten erfindungsgemäßen polaren und unpolaren superparamagnetischen Flüssigkeiten auf der Basis der Feststoffteilchen I hervorragend geeignet für Anwendungen auf den Gebieten der Wellenabdichtung, insbesondere in der Computer- und Vakuumtechnik; der Dämpfung von Lautsprechern und Schrittmotoren in der Elektrotechnik; der Dichtetrennung von feststoffen wie Erzen oder Metallen, insbesondere im Bergbau oder der chemischen Industrie; der Mikroverkapselung von Wirkstoffen, insbesondere in der Medizin oder im Pflanzenschutz; der Ölpestbeseitigung; der Energiekonversion allgemein; der Flüssigkristallanzeigen; der magnetischen Ventile, insbesondere in der Hoch- und Ultrahochvakuumtechnik; oder der Treibstoffzusätze, insbesondere in der Weltraumtechnik; oder der Markierungstechnik, insbesondere in der Medizin, der Biochemie, der Mikrobiologie oder der Biotechnologie.

<u>Beispiele</u>

In den Beispielen und Vergleichsversuchen wurde die Sättigungsmagnetisierung $M_s$ ($nTm^3/g$) sowohl der superparamagnetischen Feststoffteilchen als auch der superparamagnetischen Flüssigkeiten in einem Magnetfeld der Stärke 160 kA/m direkt nach der Herstellung bestimmt. Im Falle superparamagnetischer Flüssigkeiten, welche man nach unterschiedlichen Verfahren, aber ausgehend von den gleichen Ausgangsstoffen in den gleichen Mengen hergestellt hatte und aus denen die sedimentierbaren Feststoffteilchen

abzentrifugiert worden waren, war die Sättigungsmagnetisierung $M_s$ ein direktes Maß für den Erfolg der jeweils angewandten Herstellverfahren.

Daneben wurde noch in einigen Fällen die Ausbeute in % an tensidbeschichteten superparamagnetischen Feststoffteilchen über das Auswiegen der abzentrifugierten sedimentierbaren Feststoffteilchen bestimmt, wobei die theoretische Ausbeute zu 100 % angesetzt wurde.

Die innere Oberfläche ($m^2/g$) isolierter und getrockneter superparamagnetischer Feststoffteilchen wurde nach der BET-Methode gemessen. Die innere Oberfläche diente als Maß für die Teilchengröße.

Die Stabilität der polaren und unpolaren superparamagnetischen Flüssigkeiten wurde wie folgt ermittelt:

Man lagerte die superparamagnetischen Flüssigkeiten 7 Tage lang bei Raumtemperatur in senkrecht stehenden Röhrchen der Höhe 140 mm und des Durchmessers 3 mm. Nach dieser Zeit wurde mit Hilfe der Induktionsmethode geprüft, ob sich in den senkrecht stehenden Flüssigkeitssäulen ein Gradient der Sättigungsmagnetisierung $M_s$ gebildet hatte. Kam es nun zu einer Abnahme der Sättigungsmagnetisierung $M_s$ in den oberen Bereichen der Flüssigkeitssäulen und zu einer Zunahme der Sättigungsmagnetisierung $M_s$ in den unteren Bereichen, dann zeigte dies irreversible Veränderungen, begleitet von Sedimentation, in den betreffenden superparamagnetischen Flüssigkeiten an, d.h., die betreffenden superparamagnetischen Flüssigkeiten waren instabil. Das Ausmaß der Instabilität wurde anhand der nach der Induktionsmethode gemessenen Funktion "Sättigungsmagnetisierung $M_s$ = f (Höhe des Meßpunktes in der Flüssigkeitsäule)" beurteilt und mit:

1 keine Sedimentation - stabil;
2 geringfügige, gerade noch nachweisbare Sedimentation;
3 geringfügige, aber eindeutig nachweisbare Sedimentation;
4 starke Sedimentation;
5 praktisch vollständige Sedimentation;

benotet.

Die Viskosität (mPas) der superparamagnetischen Flüssigkeiten wurde mit Hilfe eines Rotationsviskosimeters bestimmt.

Beispiele 1 bis 7

Herstellung erfindungsgemäßer superparamagnetischer Feststoffteilchen I; allgemeine Herstellvorschrift:

Metallchloride wurden in der gewünschten Kombination mit dem gewünschten molaren Verhältnis in Wasser gelöst. Diese Lösung wurde unter Inertgas rasch zu einer vorgelegten in etwa stöchiometrischen Menge an wäßriger Natronlauge hinzugegeben, wobei sich Feststoffteilchen I der gewünschten Zusammensetzung und Größe bildeten. Das Reaktionsgemisch aus den Teilchen und wäßrigem Medium wurde durch Zugabe von Salzsäure auf pH 11 gebracht, wonach man das Reaktionsgemisch 30 Minuten bei Raumtemperatur stehen ließ. Danach wurde das Reaktionsgemisch neutralisiert. Die darin befindlichen Feststoffteilchen I wurden vom wäßrigen Medium durch Filtration abgetrennt, mehrmals mit Wasser gewaschen und an der Luft bei 120 °C getrocknet.

Die Tabelle 1 gibt Auskunft über die Zusammensetzung der Metallsalzlösungen und über die der erfindungsgemäßen superparamagnetischen Feststoffteilchen I.

Die Tabelle 2 faßt die an den Feststoffteilchen I ermittelten Meßwerte zusammen.

Tabelle 1

Zusammensetzung der Metallsalzlösungen vor dem Ausfällen und Zusammensetzung der daraus ausgefällten erfindungsgemäßen superparamagnetischen Feststoffteilchen I

| Bsp. Nr. | Zusammensetzung der Metallsalzlösungen, angegeben als molares Verhältnis der Metallionen $Co^{2\oplus}$ : | $Ni^{2\oplus}$ : | $Mn^{2\oplus}$ : | $Zn^{2\oplus}$ : | $Fe^{2\oplus}$ : | $Fe^{3\oplus}$ | q $\left(\frac{Fe^{2\oplus}}{Fe^{2\oplus}+Fe^{3\oplus}}\right)$ | Zusammensetzung der Feststoffteilchen I |
|---|---|---|---|---|---|---|---|---|
| 1 | - | - | 0,5 | 0,2 | 0,8 | 1,5 | 0,38 | $Mn_{0,5}Zn_{0,2}Fe_{2,3}O_{3,9}$ |
| 2 | - | - | 0,2 | 0,3 | 0,8 | 1,7 | 0,32 | $Mn_{0,2}Zn_{0,3}Fe_{2,5}O_{3,97}$ |
| 3 | - | - | 0,1 | 0,1 | 0,8 | 2,0 | 0,28 | $Mn_{0,1}Zn_{0,1}Fe_{2,8}O_{3,8}$ |
| 4 | - | - | 0,48 | 0,12 | 1,2 | 1,2 | 0,5 | $Mn_{0,48}Zn_{0,12}Fe_{2,4}O_{3,95}$ |
| 5 | - | - | 0,2 | 0,3 | 0,5 | 2,0 | 0,2 | $Mn_{0,2}Zn_{0,3}Fe_{2,5}O_{4}$ |
| 6 | 0,1 | - | 0,2 | 0,2 | 0,7 | 1,8 | 0,27 | $Mn_{0,2}Co_{0,1}Zn_{0,2}Fe_{2,5} \cdot O_{3,7}$ |
| 7 | - | 0,1 | 0,2 | 0,2 | 0,7 | 1,8 | 0,27 | $Mn_{0,2}Ni_{0,1}Zn_{0,2}Fe_{2,5} \cdot O_{3,96}$ |

Tabelle 2

| Sättigungsmagnetisierung $M_s$ und innere Oberfläche nach BET der erfindungsgemäßen Feststoffteilchen I | | |
|---|---|---|
| Bsp. Nr. | Sättigungsmagnetisierung $M_s$ ($nTm^3/g$) | innere Oberfläche ($m^2/g$) |
| 1 | 68 | 89 |
| 2 | 72 | 92 |
| 3 | 70 | 100 |
| 4 | 66 | 64 |
| 5 | 67 | 116 |
| 6 | 75 | 67 |
| 7 | 75 | 84 |

Vergleichsversuch A

Herstellung superparamagnetischer Feststoffteilchen des Standes der Technik; Herstellvorschrift:

Nach der bei den Beispielen 1 bis 7 angegebenen allgemeinen Herstellvorschrift wurden superparamagnetische Magnetitteilchen hergestellt ($Fe_3O_4$). Dabei lag das Verhältnis q bei 0,36. Die superparamagnetischen Magnetitteilchen wiesen eine Sättigungsmagnetisierung $M_s$ von 54 $nTm^3/g$ und eine innere Oberfläche nach BET von 104 $m^2/g$ auf.

Vergleichsversuch B

Herstellung superparamagnentischer Magan-Zink-Eisen-Ferritteilchen, deren Zusammensetzung derjenigen von paramagnetischen Teilchen des Standes der Technik entspricht; Herstellvorschrift:

Nach der bei den Beispielen 1 bis 7 angegebenen allgemeinen Herstellvorschrift wurden superparamagnetische Mangan-Zink-Eisen-Ferritteilchen hergestellt, wobei das molare Verhältnis der Metallionen in der Metallsalzlösung vor dem Ausfällen bei

$Mn^{2\oplus}:Zn^{2\oplus}:Fe^{2\oplus}:Fe^{3\oplus}$ = 0,5:0,5:0,6:1,4

lag und q gleich 0,36 war. Es resultierten nicht erfindungsgemäße superparamagnetische Feststoffteilchen der Zusammensetzung $Mn_{0,5}Zn_{0,5}Fe_2O_4$. Diese Zusammensetzung entsprach derjenigen von paramagnetischen ManganZink-Eisen-Ferritteilchen des Standes der Technik, bei der bekanntermaßen ein Maximum der Sättigungsmagnetisierung $M_s$ erreicht wird. Im Gegensatz dazu wiesen die superparamagnetischen $Mn_{0,5}Zn_{0,5}Fe_2O_4$-Feststoffteilchen bei einer inneren Oberfläche von 94 $m^2/g$ eine Sättigungsmagnetisierung $M_s$ von lediglich 50 $nTm^3/g$ auf, die deutlich unterhalb derjenigen erfindungsgemäßer superparamagnetischer Feststoffteilchen I lag.

Beispiele 8 bis 11

Herstellung erfindungsgemäßer unpolarer superparamagnetischer Flüssigkeiten aus den erfindungsgemäßen superparamagnetischen Feststoffteilchen I; allgemeine Herstellvorschrift:

Der feuchte Filterkuchen aus Feststoffteilchen I, welcher nach der bei den Beispielen 1 bis 7 beschriebenen allgemeinen Herstellvorschrift erhalten wurde, wurde in Wasser aufgeschlämmt. Zu dieser Aufschlämmung wurde eine Lösung von Natriumoleat in Wasser zugegeben, wobei die Natriumoleatmenge so gewählt worden war, daß sie für die Beschichtung der Feststoffteilchen I zumindest ausreichte.

Zu dieser Dispersion beschichteter Feststoffteilchen I in wäßrigem Medium wurden mindestens ebenso viele Volumenteile Petrolether zugegeben, wie an Volumenteilen wäßrigen Mediums vorlagen. Die resultierende Mischung aus zwei flüssigen Phasen wurde durchmischt, wobei die tensidbeschichteten superparamagnetischen Feststoffteilchen I in den Petrolether überführt wurden. Danach ließ man die Mischung so lange stehen, bis sich die beiden flüssigen Phasen wieder vollständig getrennt hatten. Das feststoffreie wäßrige Medium wurde dann abgetrennt und verworfen.

Die beschichteten Feststoffteilchen I wurden nun zweimal durch Zugabe von mindestens ebenso vielen

Volumenteilen Methanol, wie an Volumenteilen Petrolether vorlagen, aus ihren Dispersionen in Petrolether ausgeflockt und jedesmal nach der Abtrennung des Petrolether/Methanol-Mediums in den gleichen Volumenteilen frischen Petrolethers redispergiert.

Anschließend gab man soviele Gewichtsteile an höher siedenden unpolaren Flüssigkeiten hinzu, wie es dem gewünschten Gehalt an beschichteten Feststoffteilchen I in der superparamagnetischen Flüssigkeit entsprach und destillierte den Petrolether ab.

Die Tabelle 3 gibt Auskunft über die Ausgangsstoffe und die Zusammensetzung der hergestellten erfindungsgemäßen superparamagnetischen Flüssigkeiten.

Die Tabelle 4 faßt die an ihnen ermittelten Meßergebnisse zusammen.

Tabelle 3

| Herstellung erfindungsgemäßer superparamagnetischer Flüssigkeiten aus erfindungsgemäßen superparamagnetischen Feststoffteilchen I | | | | |
|---|---|---|---|---|
| Bsp. Nr. | Feststoffteilchen gemäß | Ausgangsstoffe unpolare Flüssigkeiten chemische Natur: | Physikalische Eigenschaften: | superparamagnetische Flüssigkeit: Gew.% Feststoffteilchen I |
| 8 | Bsp. 2 | Alkylaromat + hydriertes Poly-α-olefin | Dichte (20 ° C): 0,87 $gcm^{-3}$ Viskosität (40 ° C): 78 mPas | 32 |
| 9 | Bsp. 2 | Poly-isobutylen | Dichte (20 ° C) : 0,89 $gcm^{-3}$ Viskosität (20 ° C): 118 mPas Siedebereich: 290 bis 390 ° C massenmittleres Molgewicht: 320 | 31 |
| 10 | Bsp. 6 | aliphatisches Mineralöl | Dichte (20 ° C): 0,78 $gcm^{-3}$ Viskosität (25 ° C): 2 mPas Siedebereich[a]: 204-247 ° C | 37,3 |
| 11 | Bsp. 7 | " | " | 44,6 |

a: bei der niedrigen Temperatur sind 5 % des Öls verdampft, bei der höheren 95 % (bei Normaldruck);

Tabelle 4

| Sättigungsmagnetisierung $M_s$ und $M_s$/Gew. % Feststoffteilchen I der erfindungsgemäßen superparamagnetischen Flüssigkeiten | | |
|---|---|---|
| Bsp. Nr. | $M_s$ ($nTm^3/g$) | $M_s$/Gew.% ($nTm^3/g \cdot$ Gew.%) |
| 8 | 26 | 0,81 |
| 9 | 24 | 0,77 |
| 10 | 29 | 0,78 |
| 11 | 35 | 0,78 |

Beispiele 12 bis 15

Herstellung erfindungsgemäßer unpolarer superparamagnetischer Flüssigkeiten nach dem entsprechenden erfindungsgemäßen Verfahren;
Herstellvorschrift.

Es wurden vier Lösungen aus 25,46 g $MnCl_2 \cdot 4H_2O$, 11,69 g $ZnCl_2$, 68,2 g $FeCl_2 \cdot 4H_2O$ und 197,06 g $FeCl_3 \cdot 6H_2O$ in jeweils 400 ml Wasser hergestellt. Diese Lösungen wurden unter Stickstoff zu jeweils 400 ml 8 n Natronlauge zugegeben. Die vier Reaktionsgemische wurden unter Stickstoff durch Zugabe weiterer Natronlauge auf pH 11 gebracht, während 30 Minuten stehen gelassen und mittels Salzsäure neutralisiert. Die in den vier Reaktionsgemischen vorliegenden Feststoffteilchen I wurden unter Stickstoff in einem Magnetfeld sedimentiert, wonach man die wäßrigen Medien abdekantierte und die Feststoffteilchen I unter Stickstoff so lange mit Wasser extrahierte, bis das Waschwasser neutral reagierte und keine Anionen darin mehr nachweisbar waren.

Alle vier Proben wiesen die erfindungsgemäße Zusammensetzung $Mn_{0,3}Zn_{0,2}Fe_{2,5}O_4$ auf.

Die vier Proben wurden unter Stickstoff in jeweils 800 ml Wasser aufgeschlämmt, wonach man diese Aufschlämmungen mit Lösungen von jeweils 25 g Ölsäure in 500 ml Natronlauge (pH 11) versetzte. Die resultierenden Mischungen wurden unter Stickstoff auf einen pH von 11 eingestellt, bei 80°C 30 Minuten lang gerührt und danach auf Zimmertemperatur abgekühlt.

Aus den so erhaltenen Dispersionen wurden die beschichteten Feststoffteilchen I durch Zugabe von jeweils 1500 ml Methanol ausgeflockt, von den Wasser/Methanol-Medien abgetrennt, mit Methanol nachgewaschen, getrocknet und in jeweils 1500 ml Petrolether redispergiert.

Aus den so erhaltenen Dispersionen wurden die beschichteten Feststoffteilchen I durch Zugabe von jeweils 3000 ml Methanol ausgeflockt, von den Petrolether/Methanol-Medien abgetrennt, getrocknet und in jeweils 1500 ml Petrolether redispergiert.

Aus diesen vier Dispersionen wurden die sedimentierbaren Feststoffteilchen durch Zentrifugieren entfernt.

Anschließend wurden zu diesen vier Dispersionen jeweils 318,33 g jeweils einer höher siedenden unpolaren Flüssigkeit zugegeben, wobei deren Menge jeweils auf einen theoretischen Feststoffgehalt der vom Petrolether befreiten superparamagnetischen Flüssigkeiten von 30 Gew.% bezogen war.

Danach wurde der Petrolether durch Abdampfen im Vakuum aus den Dispersionen entfernt, wonach vier superparamagnetische Flüssigkeiten auf der Basis höher siedender unpolarer flüssiger Medien resultierten, die erneut zentrifugiert wurden.

Als höher siedende unpolare Flüssigkeiten wurden verwendet:

In Beispiel 12:

Ein Öl aus Alkylaromaten und hydrierten Poly-$\alpha$-olefinen; physikalische Eigenschaften, Dichte (20°C): 0,86 $gcm^{-3}$, Viskosität (40°C): 28 mPas;

in Beispiel 13:

Ein hydriertes Poly-$\alpha$-olefin-Öl; physikalische Eigenschaften, Dichte (20°C): 0,82 $gcm^{-3}$, Viskosität

(40 ° C): 15 mPas;

in Beispiel 14:

Ein Monoalkylbenzol; physikalische Eigenschaften, Siedebereich um 120 ° C bei 0,047 mbar, Dichte (20 ° C): 0,89;

in Beispiel 15:

Ein Polyisobutylenöl; physikalische Eigenschaften, Dichte (20 ° C): 0,83 $gcm^{-3}$, Viskosität (20 ° C): 118 mPas, Siedebereich (Normaldruck): 290 (5 % des Öls sind verdampft) - 390 ° C (95 % des Öls sind verdampft), massenmittleres Molgewicht: 320.

Die Tabelle 5 gibt Auskunft über Ausbeute an Feststoffteilchen I, Sättigungsmagnetisierung $M_s$ der erfindungsgemäßen superparamagnetischen Flüssigkeiten sowie deren Viskosität und deren Stabilität.

Tabelle 5

| Beispiele 12 bis 15, Versuchsergebnisse | | | | | | |
|---|---|---|---|---|---|---|
| Beispiel Nr. | Ausbeute an Feststoffteilchen I (%) | Sättigungsmagnetisierung $M_s$ der superparamagnetischen Flüssigkeiten ($nTm^3/g$) | Viskosität (mPas) bei | | | Stabilität (Note) |
| | | | 20 ° C | 40 ° C | 80 ° C | |
| 12 | >95 | 29 | 210 | 69 | 13 | 1 |
| 13 | >95 | 29 | 76 | 33 | 11 | 1 |
| 14 | >90 | 19 | 18 | 9 | 4 | 1 |
| 15 | >95 | 26 | 38 | 17 | 6 | 1 |

Vergleichsversuche C und D

Versuche zur Herstellung bekannter unpolarer superparamagnetischer Flüssigkeiten nach Ausflockungsmethoden des Standes der Technik: Vorschriften:

Bei dem Vergleichsversuch C wurde versucht, eine nach der folgenden Vorschrift hergestellt und portionierte Dispersion aus mit anionischen Tensiden beschichteten Magnetitteilchen und wäßrigem Medium mit Flüssigkeiten des Standes der Technik wie Ethanol oder Aceton (vgl. US-A- 3 843 540) auszuflocken und weiterzuverarbeiten:

Es wurde eine Lösung aus 103 g $FeCl_2 \cdot 4H_2O$ und 195 g $FeCl_3 \cdot 6H_2O$ in 400 ml Wasser hergestellt. Diese Lösung wurde unter Stickstoff zu 400 ml 8 n Natronlauge zugegeben. Das Reaktionsgemisch wurde unter Stickstoff durch Zugabe weiterer Natronlauge auf pH 11 gebracht, während 30 Minuten stehen gelassen und mittels Salzsäure neutralisiert. Die in dem Reaktionsgemisch vorliegenden Feststoffteilchen wurden unter Stickstoff in einem Magnetfeld sedimentiert, wonach man die wäßrigen Medien abdekantierte und die Feststoffteilchen unter Stickstoff so lange mit Wasser extrahierte bis das Waschwasser neutral reagierte und keine Anionen darin mehr nachweisbar waren.

Die Probe wurde unter Stickstoff in jeweils 800 ml Wasser aufgeschlämmt, wonach man diese Aufschlämmung mit einer Lösung von 25 g Ölsäure in 500 ml Natronlauge (pH 11) versetzte. Die resultierende Mischung wurde unter Stickstoff auf einen pH von 11 eingestellt, bei 80 ° C 30 Minuten lang gerührt und danach auf Zimmertemperatur abgekühlt und portioniert.

Für die Durchführung des Vergleichsversuchs D wurden die betreffenden Magnetitteilchen direkt in 1000 ml Petrolether überführt, indem man den Petrolether zu einer Portion der wäßrigen Dispersion hinzufügte, und das resultierende Gemisch aus zwei flüssigen Phasen kräftig durchmischte. Nach der Phasentrennung wurde das wäßrige Medium verworfen.

Bei dem Vergleichsversuch D wurde dann versucht, diese Dispersion von mit anionischen Tensiden beschichteten superparamagnetischen Magnetitteilchen in Petrolether mit Flüssigkeiten des Standes der Technik wie Ethanol oder Aceton (vgl. US-A- 3 843 540) auszuflocken und weiterzuverarbeiten.

In allen Fällen war aber das Redispergieren der ausgeflockten superparamagnetischen Magnetitteilchen

in Petrolether nur noch in geringem Umfang möglich. Außerdem entstanden bei diesen Vorgehensweisen so viele sedimentierbare Feststoffteilchen, daß die Versuche abgebrochen werden mußten, weil die Ausbeute an superparamagnetischen Feststoffteilchen zu gering geworden war.

Beispiele 16 bis 22

Herstellung erfindungsgemäßer polarer superparamagnetischer Flüssigkeiten nach dem entsprechenden erfindungsgemäßen Verfahren; Herstellvorschrift:

Nach der bei den Beispielen 1 bis 7 angegebenen allgemeinen Herstellvorschrift wurden sieben Proben erfindungsgemäßer Feststoffteilchen I der Zusammensetzung

$Mn_{0,3}\ Zn_{0,2}\ Fe_{2,5}\ O_4$

aus jeweils 147,1 g $FeCl_3 \cdot 6H_2O$, 65,2 g $FeCl_2 \cdot 2H_2O$, 25,5 g $MnCl_2 \cdot 4\ H_2O$ und 11,7 g $ZnCl_2$ hergestellt. Das Trockengewicht der Proben war jeweils 100 g.

Jede der sieben Proben wurde unter Stickstoff in einer Mischung aus 200 ml Wasser, 20 oder 30 g des Phosphorsäuremonoesters des ethoxylierten (p = 3 bis 5) ω-Phenylnonylalkohols und 400 ml Essigsäurecyclohexylester redispergiert und unter Rühren auf 160 °C erhitzt, wobei das Wasser abdestillierte.

Die resultierenden sieben Dispersionen aus Feststoffteilchen I und der polaren Flüssigkeit 1 wurden während 30 Minuten bei 160 °C nachgerührt. Nach dem Abkühlen wurden sie zentrifugiert, um vorhandene sedimentierbare Feststoffteilchen abzutrennen und um die Ausbeute an Feststoffteilchen I in diesen Dispersionen zu bestimmen.

Anschließend gab man soviele Gewichtsteile an polaren Flüssigkeiten 2 hinzu, wie es dem gewünschten Gehalt an Feststoffteilchen I in den polaren superparamagnetischen Flüssigkeiten entsprach und destillierte die polare Flüssigkeit 1 im Vakuum ab.

Aus den so erhaltenen polaren superparamagnetischen Flüssigkeiten ließen sich keine weiteren sedimentierbaren Feststoffteilchen mehr abzentrifugieren.

Die Tabelle 6 gibt Auskunft über die Art und Menge der verwendeten Stoffe, die Ausbeute an Feststoffteilchen I und über die an den polaren superparamagnetischen Flüssigkeiten ermittelten Meßwerte.

Tabelle 6

Beispiele 16 bis 22: Stoffe und Versuchsergebnisse

| Beispiel Nr. | saurer Phosphorsäureester (g) | Ausbeute an Feststoffteilchen I in der polaren Flüssigkeit 1 (%) | polare superparamagnetische Flüssigkeit: Gew.% Feststoffteilchen I | polare superparamagnetische Flüssigkeit: polare Flüssigkeit 2 | Sättigungsmagnetisierung (nTm$^3$/g) | Viskosität bei 20°C (mPas) | Stabilität (Note) |
|---|---|---|---|---|---|---|---|
| 16 | 20 | 70,5 | 37,5 | Phthalsäurediisodecylester | 24 | - | 1 |
| 17 | 30 | 80 | 37,5 | Adipinsäuredinonylester | 22,5 | 44,1 | 1 |
| 18 | 30 | 80,2 | 37,5 | Adipinsäurediisodecylester | 23 | 59,4 | 1 |
| 19 | 30 | 79,8 | 44,4 | Adipinsäurediisodecylester | 26,7 | 75,4 | 1 |
| 20 | 30 | 79,5 | 54,4 | Adipinsäurediisodecylester | 31 | 121 | 1-2 |
| 21 | 30 | 81 | 37,5 | Phthalsäurediisodecylester | 25,3 | 262 | 1 |
| 22 | 30 | 80,5 | 54,4 | Phthalsäurediisodecylester | 33,6 | 500 | 1-2 |

Vergleichsversuch E

Herstellung einer bekannten polaren superparamagnetischen Flüssigkeit nach einem Verfahren des Standes der Technik (US-PS 4 430 239); Herstellvorschrift:

Nach der in Beispiel 1 der US-PS 4 430 239 angegebenen Vorschrift wurden ca. 100 g an superparamagnetischem Magnetit hergestellt und in 313 g des sauren Phosphorsäuremonoesters des ethoxylierten (p = 3 bis 5) ω-Phenylnonylalkohols und 3130 ml Wasser dispergiert.

Zu dieser Dispersion wurden 3000 ml Aceton zugegeben, wodurch die beschichteten superparamagnetischen Magnetitteilchen ausgeflockt wurden. Die ausgeflockten Teilchen wurden von der Wasser/Aceton-Mischung abgetrennt und mit 6000 ml Aceton gewaschen.

Die gewaschenen, feuchten Teilchen wurden in 250 ml Azelainsäuredi-(2-ethylhexyl)ester redispergiert, wonach man restliches Wasser und Aceton abdestillierte.

Es resultierte eine polare superparamagnetische Flüssigkeit mit einem Gehalt an superparamagnetischen Feststoffteilchen von 33 Gew.%. Sie wies eine Sättigungsmagnetisierung von 39 $nTm^3/g$ und eine Viskosität bei 20 °C von 140 mPas auf. Sie enthielt aber noch größere Mengen an sedimentierbaren Feststoffteilchen. Diese wurden abzentrifugiert, wodurch der Anteil an superparamagnetischen Feststoffteilchen in der polaren superparamagnetischen Flüssigkeit auf 23,5 Gew.% sank. Aber selbst diese geringer konzentrierte superparamagnetische Flüssigkeit war nicht stabil, sondern zeigte eine merkliche Sedimentation (Note 3 bis 4).

**Patentansprüche**

1. Superparamagnetische Feststoffteilchen, die der allgemeinen Formel I

   $M_vMn_wZn_xFe_yO_z$ (I)

   entsprechen, in welcher für die Variablen folgende Bedingungen gelten:

| | |
|---|---|
| M | Co und/oder Ni |
| v, w | 0 bis 0,998 |
| x | 0,001 bis 0,998 |
| y | 2,001 bis 2,998 |
| z | 3,001 bis 4 |
| v + w + x | 0,002 bis 0,999 |
| v + w + x + y | 3 |
| $v \neq 0$ | wenn w = 0 |
| $w \neq 0$ | wenn v = 0 |

2. Polare und unpolare superparamagnetische Flüssigkeiten, welche mit Tensiden beschichtete superparamagnetische Feststoffteilchen I gemäß Anspruch 1 in flüssigen Medien kolloidal dispergiert enthalten.

3. Unpolare superparamagnetische Flüssigkeiten nach Anspruch 2, welche mit anionischen Tensiden beschichtete superparamagnetische Feststoffteilchen I gemäß Anspruch 1 in unpolaren flüssigen Medien dispergiert enthalten.

4. Polare superparamagnetische Flüssigkeiten nach Anspruch 2, welche mit sauren Phosphorsäureestern beschichtete superparamagnetische Feststoffteilchen I gemäß Anspruch 1 in polaren flüssigen Medien kolloidal dispergiert enthalten.

5. Verfahren zur Herstellung unpolarer superparamagnetischer Flüssigkeiten gemäß Anspruch 3 aus superparamagnetischen Feststoffteilchen I gemäß Anspruch 1 und unpolaren flüssigen Medien durch Ausfällung der Teilchen aus wäßrigen Metallsalzlösungen, welche der chemischen Zusammensetzung der Teilchen entsprechen, mittels einer Base, Beschichtung der Teilchen mit einem anionischen Tensid und Überführung der Teilchen in eine unpolare Flüssigkeit, dadurch gekennzeichnet, daß man
   a) die in dem wäßrigen Medium befindlichen beschichteten Teilchen I mit Methanol ausflockt, abtrennt und in einer unpolaren Flüssigkeit redispergiert
   und/oder
   b) die bereits auf beliebige Weise in einem unpolaren flüssigen Medium dispergierten beschichteten Teilchen I mit Methanol ausflockt, abtrennt und in einer unpolaren Flüssigkeit redispergiert.

6. Verfahren nach Anspruch 5, dadurch gekennzeichnet, daß man die Variante b zwei- oder mehrmals wiederholt.

**7.** Verfahren nach Anspruch 5 oder 6, dadurch gekennzeichnet, daß man die hiernach erhaltenen Dispersionen zunächst mit einer wäßrigen Base und danach solange mit Wasser wäscht, bis das Waschwasser neutral reagiert und keine Anionen mehr darin nachweisbar sind.

**8.** Verfahren zur Herstellung unpolarer superparamagnetischer Flüssigkeiten aus superparamagnetischen Feststoffteilchen I gemäß Anspruch 1 und unpolaren flüssigen Medien nach Anspruch 3, durch Ausfällung der Teilchen aus wäßrigen Metallsalzlösungen, welche der chemischen Zusammensetzung der Teilchen entsprechen, mittels einer Base, Beschichtung der Teilchen mit einem anionischen Tensid und Überführung der Teilchen in eine unpolare Flüssigkeit, dadurch gekennzeichnet, daß man die hiernach erhaltenen Dispersionen zunächst mit einer wäßrigen Base und danach solange mit Wasser wäscht, bis das Waschwasser neutral reagiert und keine Anionen mehr darin nachweisbar sind.

**9.** Verfahren zur Herstellung polarer superparamagnetischer Flüssigkeiten gemäß Anspruch 4 aus superparamagnetischen Feststoffteilchen I gemäß Anspruch 1 und polaren flüssigen Medien durch Ausfällen der Teilchen aus wäßrigen Metallsalzlösungen, welche der chemischen Zusammensetzung der Teilchen entsprechen, mittels einer Base, Beschichtung der Teilchen mit einem sauren Phosphorsäureester und Überführung der Teilchen aus dem wäßrigen Medium in eine polare Flüssigkeit, welche höher siedet als Wasser, dadurch gekennzeichnet, daß man hierbei

a') die beschichteten superparamagnetischen Feststoffteilchen in eine zwischen 110 und 250 °C siedende polare Flüssigkeit 1 überführt und daß man

b') die resultierende Dispersion nach dem Abdestillieren des Wassers mit einer polaren Flüssigkeit 2 versetzt, welche höher siedet als die Flüssigkeit 1, wonach man die Flüssigkeit 1 aus der Dispersion entfernt.

## Claims

**1.** Superparamagnetic solid particles which are of the formula I

$M_vMn_wZn_xFe_yO_z$ (I)

where
M is Co or Ni,
v and w are each from 0 to 0.998,
x is from 0.001 to 0.998,
y is from 2.001 to 2.998,
z is from 3.001 to 4,
$v + w + x$ is from 0.002 to 0.999,
$v + w + x + y$ is 3,
$v \neq 0$ when $w = 0$ and
$w \neq 0$ when $v = 0$.

**2.** A polar or nonpolar superparamagnetic liquid which contains surfactant-coated superparamagnetic solid particles I as claimed in claim 1 as a colloidal dispersion in a liquid medium.

**3.** A nonpolar superparamagnetic liquid as claimed in claim 2, which contains superparamagnetic solid particles I as claimed in claim 1, coated with anionic surfactants, as a dispersion in a nonpolar liquid medium.

**4.** A polar superparamagnetic liquid as claimed in claim 2, which contains superparamagnetic solid particles I as claimed in claim 1, coated with acidic phosphoric esters, as a colloidal dipsersion in a polar liquid medium.

**5.** A process for the preparation of a nonpolar superparamagnetic liquid as claimed in claim 3 from superparamagnetic solid particles I as claimed in claim 1 and a nonpolar liquid medium by precipitating the particles from aqueous metal salt solutions which correspond to the chemical composition of the particles by means of a base, coating the particles with an anionic surfactant and transferring the particles to a nonpolar liquid, wherein

a) the coated particles I present in the aqueous medium are flocculated with methanol, separated off

and redispersed in a nonpolar liquid
or
b) the coated particles I already dispersed by any method in a nonpolar liquid medium are flocculated with methanol, separated off and redispersed in a nonpolar liquid.

6. A process as claimed in claim 5, wherein variant b is repeated two or more times.

7. A process as claimed in claim 5 or 6, wherein the resulting dispersion is washed first with an aqueous base and then with water until the wash water is neutral and anions are no longer detectable therein.

8. A process for the preparation of a nonpolar superparamagnetic liquid from superparamagnetic solid particles I as claimed in claim 1 and a nonpolar liquid medium as claimed in claim 3 by precipitating the particles from aqueous metal salt solutions which correspond to the chemical composition of the particles by means of a base, coating the particles with an anionic surfactant and transferring the particles to a nonpolar liquid, wherein the resulting dispersion is washed first with an aqueous base and then with water until the wash water is neutral and anions are no longer detectable therein.

9. A process for the preparation of a polar superparamagnetic liquid as claimed in claim 4 from superparamagnetic liquid particles I as claimed in claim 1 and a polar liquid medium by precipitating the particles from aqueous metal salt solutions which correspond to the chemical composition of the particles by means of a base, coating the particles with an acidic phosphoric ester and transferring the particles from the aqueous medium to a polar liquid which has a higher boiling point than water wherein
a') the coated superparamagnetic solid particles are transferred to a polar liquid 1 which boils in the range from 110 to 250 ° C, and
b') a polar liquid 2 which has a higher boiling point than the liquid 1 is added to the resulting dispersion after the water has been distilled off, and the liquid 1 is then removed from the dispersion.

**Revendications**

1. Particules de matières solides surperparamagnétiques répondant à la formule générale I

$M_vMn_wZn_xFe_yO_z$ (I)

dans laquelle les symboles ont les significations suivantes :
M représente Co et/ou Ni
v, w sont des nombres allant de 0 à 0,998
x est un nombre allant de 0,001 à 0,998
y est un nombre allant de 2,001 à 2,998
z est un nombre allant de 3,001 à 4
la somme v + w + x a une valeur de 0,002 à 0,999
la somme v + w + x + y est égale à 3
v est différent de 0 lorsque w est égal à 0
w est différent de 0 lorsque v est égal à 0.

2. Liquides superparamagnétique polaires et non polaires contenant en dispersion colloïdale dans des milieux aqueux des particules de matières solides superparamagnétiques I de la revendication 1 revêtues d'agents tensioactifs.

3. Liquides superparamagnétiques non polaires selon la revendication 2, contenant en dispersion dans des milieux liquides non polaires des particules de matières solides superparamagnétiques I de la revendication 1 revêtues par des agents tensioactifs anioniques.

4. Liquides superparamagnétiques polaires selon la revendication 2, contenant en dispersion colloïdale dans des milieux liquides polaires des particules de substances solides superparamagnétiques de la revendication 1 revêtues d'esters phosphoriques acides.

5. Procédé de préparation des liquides superparamagnétiques non polaires de la revendication 4 à partir

de particules de matières solides superparamagnétiques I de la revendication 1 et de milieux liquides non polaires par précipitation des particules à partir de solutions aqueuses de sels métalliques correspondant à la composition chimique des particules, à l'aide d'une base, revêtement des particules par un agent tensioactif anionique et transfert des particules dans un liquide non polaire, caractérisé en ce que :

a) on flocule par le méthanol, les particules revêtues I qui se trouvent dans le milieu aqueux, on les sépare et on les redisperse dans un liquide non polaire, et/ou

b) on flocule par le méthanol les particules revêtues I déjà dispersées par un moyen quelconque dans un milieu liquide non polaire, on les sépare et on les redisperse dans un liquide non polaire.

**6.** Procédé selon la revendication 5, caractérisé en ce que l'on répète deux ou plusieurs fois les opérations de la variante b).

**7.** Procédé selon la revendication 5 ou 6, caractérisé en ce que l'on lave les dispersions obtenues, d'abord par une base aqueuse puis à l'eau jusqu'à ce que les eaux de lavage aient une réaction neutre et ne contiennent plus d'anions.

**8.** Procédé de préparation de liquides superparamagnétiques non polaires à partir de particules de substances solides superparamagnétiques I de la revendication 1 et de milieux liquides non polaires selon la revendication 3, par précipitation des particules à partir de solutions aqueuses de sels métalliques correspondant à la composition chimique des particules, à l'aide d'une base, revêtement des particules par un agent tensioactif anionique et transfert des particules dans un liquide non polaire, caractérisé en ce que l'on lave les dispersions ainsi obtenues d'abord par une base aqueuse puis à l'eau jusqu'à ce que les eaux de lavage aient une réaction neutre et ne contiennent plus d'anions.

**9.** Procédé de préparation de liquides superparamagnétiques polaires selon la revendication 4 à partir de particules de substances solides superparamagnétiques I de la revendication 1 et de milieux liquides polaires par précipitation des particules à partir de solutions aqueuses de sels métalliques correspondant à la composition chimique des particules, à l'aide d'une base, revêtement des particules par un ester phophorique acide et transfert des particules du milieu aqueux dans un liquide polaire bouillant plus haut que l'eau, caractérisé en ce que :

a') on transfère les particules de substances solides superparamagnétiques revêtues dans un liquide polaire 1 bouillant entre 110 et 250 degrés C, et

b') après distillation de l'eau de la dispersion ainsi obtenue, on ajoute un liquide polaire 2 bouillant plus haut que le liquide 1, après quoi on élimine le liquide 1 de la dispersion.